# EUROPÄISCHE PATENTSCHRIFT

(11) **EP 3 167 873 B1**
(45) Veröffentlichungstag und Bekanntmachung des Hinweises auf die Patenterteilung: **10.04.2019**
(21) Anmeldenummer: 15194145.7
(22) Anmeldetag: 11.11.2015
(51) Int. Cl.: A61K 9/08, A61K 31/549, A61P 31/04

(54) **WÄSSRIGE LÖSUNG VON KONDENSATIONSPRODUKTEN VON TAURINAMID UND METHYLENGLYKOL ZUR VERWENDUNG ALS ANTIINFEKTIVES MITTEL BEI DER IMPLANTATION VON VORRICHTUNGEN IN DER HERZCHIRURGIE**
AQUEOUS SOLUTION OF CONDENSATION PRODUCTS OF TAURINAMIDE AND METHYLENE GLYCOL FOR USE AS ANTI-INFECTIVE AGENT IN THE IMPLANTATION OF DEVICES IN CARDIAC SURGERY
SOLUTION AQUEUSE DE PRODUITS DE CONDENSATION DE TAURINAMIDE ET DE METHYLENE GLYCOL EN TANT QU'AGENT ANTI-INFECTIEUX LORS DE L'IMPLANTATION DE DISPOSITIFS DANS LA CHIRURGIE CARDIAQUE

(43) Veröffentlichungstag der Anmeldung: 17.05.2017
(73) Patentinhaber: Weis, Christian Edwin, 97297 Waldbrüttelbrunn (DE); Herdeis, Claus, 80809 München (DE)
(72) Erfinder: Weis, Christian Edwin, 97297 Waldbrüttelbrunn (DE); Herdeis, Claus, 80809 München (DE)
(74) Vertreter: Friese Goeden Patentanwälte PartGmbB

(56) Entgegenhaltungen:
- US-A1- 2013 084 319
- OBRADOVIC B ET AL: "Taurolidin as a local disinfectant in open heart surgery", ISRAEL JOURNAL OF MEDICAL SCIENCES, Bd. 32, Nr. 10, 1996, Seite 1020, XP009188673, & NINTH ANNUAL MEETING OF THE MEDITERRANEAN ASSOCIATION OF CARDIOLOGY AND CARDIAC SURGERY; TEL-AVIV, ISRAEL; OCTOBER 20-23, 1996 ISSN: 0021-2180
- G. MARSCH ET AL: "Prevention of pacemaker infections with perioperative antimicrobial treatment: an in vitro study", EUROPACE, Bd. 16, Nr. 4, 8. August 2013 (2013-08-08) , Seiten 604-611, XP055252187, GB ISSN: 1099-5129, DOI: 10.1093/europace/eut222

## Beschreibung

Die vorliegende Erfindung betrifft die Verbesserung des therapeutischen Erfolgs von Implantationen in der Herzchirurgie, insbesondere von Implantationen von medizinischen Vorrichtungen, die unter dem Begriff "implantierbare elektronische Vorrichtungen für die Behandlung von kardiovaskulären Erkrankungen" zusammengefasst werden. In der einschlägigen medizinischen Fachliteratur, die überwiegend englischsprachig ist, werden für diese Vorrichtungen in aller Regel die Sammelbegriffe "cardiac-implantable electrophysiological devices" oder "cardiovascular implantable electronic devices", abgekürzt CIEDs, verwendet. Auch der Begriff "cardiac rhythm management device", abgekürzt CRMD, wird verwendet.

Vorrichtungen, die unter einem dieser Begriffe zusammengefasst werden, umfassen, ohne dass die folgende Auflistung Anspruch auf Vollständigkeit erhebt, verschiedene Typen von Herzschrittmachern (pacemaker), implantierbare Kardioverter-Defibrillatoren (implantable cardioverterdefibrillators, abgekürzt ICDs), kardiale Resynchronisationstherapie-Defibrillatoren (cardiac resynchronization therapy-defibrillator, abgekürzt CRT-D), subkutan implantierbare Kardioverter-Defibrillatoren (subcutaneous implantable cardioverter-defibrillator, abgekürzt S-ICD) und implantierte Überwachungsvorrichtungen für diagnostische Zwecke.

Da sich die vorteilhaften klinischen Resultate, die durch die Anwendung der Erfindung der vorliegenden Anmeldung erzielt werden, bisher vor allem auf dem Gebiet der chirurgischen Implantation von Herzschrittmachern zeigen ließen, werden die Erfolge und die praktische Durchführung der Erfindung in der folgenden Beschreibung unter Bezug auf die Implantation von Herzschrittmachern erläutert.

Herzschrittmacher dienen dazu, Herzrhythmusstörungen, z.B. eine Verlangsamung der Herztätigkeit (Bradykardie), durch elektrische Stimulation des Herzens zu beheben. Herzschrittmacher (pacemaker) gibt es in Abhängigkeit vom Hersteller und vom speziellen Einsatzzweck in verschiedenen Bauarten (z.B. mit einer oder mehreren Elektroden oder Sonden, wobei man unipolare oder bipolare Elektroden, sogenannte Ankerelektroden, Schraubenelektroden, gerade oder vorgebogene Elektrode und VDD-Elektroden kennt).

Die Grundelemente eines Herzschrittmachers umfassen ein Gehäuse (oder Aggregat), das heute in der Regel aus Edelstahl oder Titan hergestellt ist und die zur Erzeugung der elektrischen Impulse benötigten Batterien und Elektronikeinrichtungen enthält und Anschlusselemente für eine oder mehrere Elektroden oder Sonden aufweist. Das Gehäuse ist hermetisch versiegelt, z.B. durch Laserschweißen, und weist sehr glatte, polierte Oberflächen auf. Über die Elektroden oder Sonden wird die Verbindung zum Herzen hergestellt und werden Stimulationsimpulse abgegeben und EKG-Signale überwacht.

Zur Verbindung der in die Herzkammer und/oder den Vorhof eingeführten inerten Elektroden mit dem Gehäuse des Herzschrittmachers dienen Elektrodenkabel mit einem metallischen Leiter, der von einer Isolation aus Kunststoff, in der Regel einem Polyurethan, umgeben ist.

Die Gehäuse von Herzschrittmachern weisen etwa die Größe einer großen Münze oder einer Zündholzschachtel auf.

Herzschrittmacher werden im Rahmen eines kleineren chirurgischen Eingriffs eingesetzt. Dabei werden die Elektroden oder Sonden ohne Vollnarkose mit dissoziativer Anaesthesie durch einen Hautschnitt über eine Vene ins Herz (zum rechten Herz, Vorhof und ggf. auch Kammer) vorgeschoben. Danach, wenn sie korrekt positioniert sind, wird das Gehäuse (Aggregat) des Herzschrittmachers mit den Sonden verbunden und in einer Gewebetasche (pocket), meist in der Brustwand unterhalb des Schlüsselbeins, implantiert.

Durch die Entwicklung und den Einsatz von CIEDs, z.B. Herzschrittmachern, konnte erreicht werden, dass sich die Morbiditäts- und Mortalitätskennwerte aufgrund von Herzerkrankungen in der Bevölkerung erheblich verbessert haben. Die Zahl der Implantationen von CIEDs wie z.B. Herzschrittmachern ist daher in den letzten Jahrzehnten bzw. Jahren erheblich gestiegen.

Dabei wurde allerdings festgestellt, dass sich gleichzeitig bestimmte Probleme, die auf Infektionen im Bereich der implantierten Vorrichtungen zurück zu führen sind, verschärft haben, und zwar verglichen mit der Steigerung der Zahl der implantierten CIEDs überproportional. Die Probleme werden als vorrichtungsbedingte Infektionen oder devicerelated infections, abgekürzt DRIs, bezeichnet.

In einer Reihe von Veröffentlichungen der jüngeren Zeit befassen sich verschiedene Autoren mit DRIs und ihrer Verhinderung und Therapie. Es können hier beispielhaft genannt werden die Veröffentlichungen von Dong Heun Lee et al., "Differences in Mortality Rates between Pocket and Nonpocket Cardiovascular Implantable Electronic Device Infection", in: PACE 2015; 00:1-8; Gareth J. Padfield et al., "Preventing cardiac implantable electronic device infections", Heart Rhythm 2015; 0:-2-13; Martin Rohacek et al., "Cardiovascular implantable electronic device infections: associated risk factors and prevention", Swiss Med Wkly. 2015; 145:w15157; Avirup Guha et al., "Cardiac implantable device infection in patients with end-stage renal disease", Heart Rhythm 2015; 0:0-7; Achana Kodali et al., "A case of Late Implantable Cardiac Device Infection with Aspergillus in an Immunocompetent Host", A J Case Rep, 2015; 16: 520-523; Jonathan A. T. Sandoe et al., "Guidelines for the diagnosis, prevention and management of implantable cardiac electronic device infection. Report of a joint Working Party project on behalf of the British Society for Antimicrobial Chemotherapy (BSAC, host organization), British Heart Valve Society (BHVS) and British Society for Echocardiography (BSE)", J. Antimicrob. Chemother 2015; 70: 325-359.

In der Veröffentlichung J. Alberto Lopez, "Conservative management of infected pacemaker and implantable defibrillator sites with a closed antimicrobial irrigation system", Europace (2013), 15, 541-545, wird die Gesamtproblematik erläutert und eine Vorgehensweise für den Fall einer aufgetretenen Infektion beschrieben, und zwar eine Spülung (infusion, irrigation) der infizierten Schrittmachertasche und des entnommenen Schrittmacher-Gehäuses mit einer Antibiotika-Lösung (Vancomycin/Gentamicin-Lösung).

In den genannten Veröffentlichungen werden Überlegungen dazu angestellt, worauf die gestiegenen DRI Zahlen zurück zu führen sind, und welche Möglichkeiten es geben könnte, die Infektionszahlen zu senken, z.B. durch Änderung der Operationstechnik und/oder durch Einsatz von geeigneten Medikamenten. Für nähere Einzelheiten wird auf den Inhalt der genannten Schriften, und der darin genannten älteren Veröffentlichungen, verwiesen.

Zu den Maßnahmen, die man im Hinblick auf eine mögliche Verminderung der Infektionszahlen untersucht hat, gehört eine perioperative antibiotische Prophylaxe in Form einer intravenösen Injektion eines Antibiotikums, z.B. von Cefazolin, direkt vor dem chirurgischen Eingriff in den Patienten und ggf. noch einmal danach. Bei einem Versuch, die Infektionsrate durch Irrigation (Wundspülung) mit dem Antiseptikum Povidon-Iod (Braunol) zu verbessern, wurde keine nennenswerte Verbesserung beobachtet. Zur Infektionsreduzierung wurde auch schon ein antibakteriell infiltrierter Gewebeumschlag für die zu implantierende Vorrichtung erprobt, wobei sich zwar Verbesserungen andeuteten, die Ergebnisse jedoch noch einer weiteren Verifizierung bedürfen. Die Verwendung von Wasserstoffperoxid als antibakterielles (bakterizides) Mittel, z.B. in Form einer damit getränkten Gaze, wird ebenfalls erprobt.

In einer Veröffentlichung von Georg Marsch et al., "Prevention of pacemaker infections with perioperative antimicrobial treatment: an in vitro study", in Europace (2014), 16, 604-611, untersuchten die Autoren, ob eine antibakterielle Vorbehandlung von Herzschrittmacher-Gehäusen mit Antibiotika (Vancomycin, Daptomycin, Cefuroxim, Tazobac oder Nebacetin) oder Antiseptika (Povidon-Iod oder Octenidindihydrochlorid) eine Maßnahme zur Verminderung der Zahl von vorrichtungsbedingten Infektionen (DRIs) darstellen könnte. Zu diesem Zweck prüften sie in vitro die Wirkung von Lösungen von Antibiotika und Antiseptika auf Titanplättchen (0,4 cm²), die sie durch Schneiden von Herzschrittmachergehäusen aus Titan erzeugten. Insbesondere das Antibiotikum Nebacetin lieferte vielversprechende Ergebnisse, während andere Antibiotika oder die beiden getesteten Antiseptika entweder keine überzeugende antibakterielle Wirkungen erkennen ließen oder sich als zellschädigend erwiesen, was im Hinblick auf die Wundheilung nach dem chirurgischen Eingriff einen gravierenden Nachteil darstellt.

Es besteht somit weiterhin ein Bedarf nach Maßnahmen bzw. Mitteln, die den therapeutischen Erfolg der Implantation von CIEDs dadurch verbessern, dass das Auftreten von vorrichtungsbedingten Infektionen (DRIs) reduziert oder vermieden werden kann.

Die Erfindung wird durch die beigefügten Patentansprüche in Verbindung mit der nachfolgenden detaillierteren Erläuterung verschiedener ihrer Aspekte näher beschrieben.

Die Aufgabe wird erfindungsgemäß durch eine wässrige Lösung für eine Verwendung gemäß Anspruch 1 gelöst. Vorteilhafte Weiterbildungen der Erfindung finden sich in den Unteransprüchen 2 bis 5.

Die Erfindung betrifft die Verwendung einer wässrigen Lösung von Kondensationsprodukten von Taurinamid und Methylenglykol und deren Salzen, insbesondere von Taurolidin, Taurultam oder Cyclotaurolidin, als antiinfektives Mittel bei der Implantation von implantierbaren Vorrichtungen in der Herzchirurgie, insbesondere bei der Implantation einer implantierbaren elektronischen Vorrichtung, die ausgewählt ist aus Herzschrittmachern, implantierbaren Kardioverter-Defibrillatoren, kardialen Resynchronisationstherapie-Defibrillatoren, subkutan implantierbaren Kardioverter-Defibrillatoren und implantierbaren elektronischen Überwachungsvorrichtungen für diagnostische Zwecke.

Die verwendete wässrige Lösung enthält als antimikrobielle Wirksubstanz Taurolidin und/oder dessen Hydrolyseprodukte in einer Konzentration von 0,5 bis 3 Gew.-% oder gelöstes Taurultam in einer Konzentration von 1 bis 7,5 Gew.-%, sowie außerdem als Lösungsvermittler für die antimikrobielle Wirksubstanz und/oder als die Benetzung von Metall- und Kunststoffoberflächen förderndes Mittel ein wassermischbares, physiologisch verträgliches Polymer. Das wassermischbare Polymer ist vorzugsweise Polyvinylpyrrolidon (PVP, Povidon) mit einem mittleren Molekulargewicht von weniger als 30.000, vorzugsweise weniger als 10.000, beispielsweise zwischen 7000 und 9000, und ist in der Lösung vorzugsweise in Mengen von 3 bis 7 Gew.-%, beispielsweise von 5 Gew.-%, vorhanden.

Bei der Verwendung wird üblicherweise so vorgegangen, dass die implantierbare Vorrichtung vor der Implantation für die zur Erzielung der gewünschten optimalen antimikrobiellen Wirkung und zur vollständigen Benetzung aller Oberflächen der Vorrichtung erforderliche Zeit, gemäß einer bevorzugten Praxis etwa 20 Minuten, in die Lösung eingelegt wird und danach die implantierbare Vorrichtung, z.B. das Gehäuse eines Herzschrittmachers, mit einem Film der wässrigen Lösung auf ihrer Oberfläche in die vom operierenden Chirurgen vorbereitete Tasche implantiert wird.

Die Tasche wird anschließend mit der restlichen Lösung (ca. 100 ml) gespült.

Die Anwendung der Erfindung führt - wie nachfolgende unter Bezugnahme auf Ergebnisse der klinischen Erprobung gezeigt wird - zur Verbesserung des therapeutischen Erfolgs und zur Reduzierung von Infektionen bei der Implantation von implantierbaren elektronischen Vorrichtungen in der Herzchirurgie.

Mit dem Begriff "Kondensationsprodukte von Taurinamid und Methylenglykol und deren Salze" werden Verbindungen bezeichnet, die als prominentesten Vertreter die Verbindung Taurolidin umfassen, für das eine besonders vorteilhafte Herstellung in hochreiner Form ohne unerwünschte Nebenprodukte beschrieben ist im Europäischen Patent EP 0 863 133 B1 bzw. dem entsprechenden US-Patent US 5,889,189.

Bei der Verbindung Taurolidin liegt das molekulare Verhältnis von Taurinamid zu Methylenglykol (Methandiol, Formaldehydhydrat) bei 2 : 3 bzw bei 1 : 1,5. Es ist bekannt, dass Taurolidin bei der Auflösung in einem wässrigen Medium hydrolysiert, so dass man Schwierigkeiten hat, Taurolidin in der wässrigen Lösung als solches nachzuweisen. Es wird davon ausgegangen, dass die wässrige Lösung vor allem die Verbindungen Taurultam und Methyloltaurultam enthält, bei denen das Verhältnis von Taurinamid zu Methylenglykol 1 : 1 bzw. 1 : 2 beträgt.

Statt von Taurolidin kann auch von einer als Cyclotaurolidin bezeichneten Verbindung ausgegangen werden, deren Herstellung und Eigenschaften im Europäischen Patent EP 1 882 476 bzw. dem entsprechenden US-Patent US 7,820,651 beschrieben sind. Auf den gesamten Inhalt der genannten Patente, in denen auch die Kondensation von Taurinamid und Methylenglykol bei unterschiedlichen Molverhältnissen der Partner der Kondensationsreaktion genauer beschrieben wird, wird zur Ergänzung der vorliegenden Beschreibung ausdrücklich verwiesen.

Da Taurolidin das bekannteste und in der Praxis wichtigste Kondensationsprodukt von Taurinamid und Methylenglykol ist und seine Bezeichnung manchmal sogar mit der ganzen Gruppe von Kondensationsprodukten gleichgesetzt wird, wird die Erfindung nachfolgend unter Verwendung des Begriffs "Taurolidin" für die antibakterielle Wirksubstanz beschrieben. Eine Beschränkung der Lehre der Erfindung durch eine Beschreibung unter Bezugnahme auf "Taurolidin" ist dabei aber nicht beabsichtigt.

Taurolidin ist dafür bekannt, dass seine antimikrobielle Wirkung auf einem Transfer von Methylolgruppen auf Zellwandkomponenten von Bakterien beruht. Es ist auch bekannt, dass Taurolidin seine volle antibakterielle Wirkung erst bei einer Kontaktdauer seiner Lösung mit den Mikroorganismen, z.B. mit mit Bakterien kontaminierten Oberflächen, von einigen Minuten entwickelt, und zwar typischerweise innerhalb von etwa 10 Minuten oder mehr. Dabei hat es sich auch als wirksam gegen Biofilme erwiesen, gegen die andere Antiseptika oder Antibiotika keine oder eine nur schlechte Wirksamkeit zeigen.

Es wurde bisher noch nichts von einer Entwicklung von Resistenzen gegen Taurolidin berichtet, was einen großen Vorteil gegenüber einer Verwendung von Antibiotika darstellt, bei deren Verwendung in der Regel nach einer gewissen Zeit Resistenzprobleme auftreten. Es hat sich ferner gezeigt, dass Taurolidin auch gegen die problematischen (multi)resistenten nosokomialen Erreger, wie z.B. MRSA (Methicillin-resistenter Staphylococcus aureus) und VRE (Vancomycin-resistente Enterokokken) und andere, unter dem Sammelbegriff ESKAPE Pathogene geführte Keime voll wirksam ist.

Wässrige Lösungen von Taurolidin sind bekannt und werden im Gesundheitswesen bereits erfolgreich verwendet. Eine besonders wichtige Verwendung derartiger Lösungen ist diejenige als Medizinprodukte in Form sogenannter "Locklösungen", mit denen Katheter für die Zeit von Verwendungspausen, z.B. zwischen Hämodialysebehandlungen oder in Pausen bei der Zufuhr von Medikamenten oder Nährlösungen, gefüllt werden. Nähere Einzelheiten zu dieser Art von Verwendung finden sich in den bereits oben genannten Patenten EP 0 863 133 und EP 1 882 476 sowie außerdem in den Patenten EP 1 089 738 sowie EP 1 814 562, auf deren Inhalt an näheren Einzelheiten Interessierte verwiesen werden. Ein Produktsortiment für die Verwendung als Locklösung stellen beispielsweise die unter der Marke TauroLock® im Handel erhältlichen Produkte der TauroPharm GmbH dar.

Außerdem werden wässrige Lösungen von Taurolidin auch als Arzneimittel eingesetzt, und zwar als Instillationslösungen und chirurgische Spüllösungen mit Taurolidingehalten von 0,5 bzw. 2,0 Gew.-%, die als Lösungsvermittler außerdem Polyvinylpyrrolidon enthalten. Derartige Produkte sind die unter der Marke Taurolin® vertriebenen Produkte (Taurolin® (Taurolidin "Geistlich") 2% Instillationslösung; Taurolin® (Taurolidin "Geistlich") 0,5% chirurgische Spüllösung; Taurolin® Ringer 0,5%) sowie das Produkt Taurolidin Nova 2,0% der TauroPharm GmbH.

Taurolidin Nova 2,0% ist laut Gebrauchsinformation deklariert als Breitband-Chemotherapeutikum für eine Verwendung bei ausgedehnten eitrigen Entzündungen des Bauchfells, die meist durch Mikroorganismen wie Bakterien aus dem Magen-Darm-Trakt, dem Stuhl und den ableitenden Harnwegen hervorgerufen werden (lokale oder diffuse Peritonitis purulenter oder sterkoraler Genese). Das Produkt enthält in 1 ml Lösung 20 mg Taurolidin in Wasser für Injektionszwecke, und als sonstige Bestandteile Natriumhydroxid-Lösung zur pH-Wert-Einstellung und Povidon.

Die nachfolgend beschriebenen Ergebnisse der neuen klinischen Verwendung im Sinne der vorliegenden Erfindung wurden erhalten unter Verwendung einer Lösung mit 2 Gew.-% Taurolidin.

### Klinische Ergebnisse:

In einer kardiologischen Station eines akademischen Lehrkrankenhauses, in der routinemäßig und in großer Zahl Patienten Herzschrittmacher implantiert werden, gehörte es zur Praxis, zur Infektionsvermeidung die zu implantierenden Herzschrittmachergehäuse während der Implantation mit einer Lösung von Wasserstoffperoxid zu behandeln, wobei gleichzeitig eine Wundspülung mit Wasserstoffperoxid stattfindet. Dabei kommt es zur Freisetzung von Sauerstoff und starkem Aufschäumen. Trotz der bakteriziden Eigenschaften von verdünntem Wasserstoffperoxid ist die Wirkung der Behandlung als Infektionsprophylaxe bei der Implantation von Geräten nicht verifiziert.

Bei der Implantation von Herzschrittmachern wurde beobachtet, dass es trotz der Wasserstoffperoxidbehandlung nach der Implantation, nach kürzerer oder längerer Zeit innerhalb des Revisionszeitraums von 2 Monaten, zu Infektionen kam. Und zwar wurde eine Infektionsrate von etwa 9 Infektionen pro 60 Implantationen bzw. 10 Infektionen pro 70 Implantationen beobachtet.

Wurde dagegen gemäß der Vorgehensweise der Erfindung verfahren, bei der der zu implantierende Herzschrittmacher vor der Implantation etwa 20 Minuten in eine Lösung von 2,0 Gew.-% Taurolidin in Wasser für Injektionszwecke eingelegt wurde, danach die implantierbare Vorrichtung, z.B. das Gehäuse eines Herzschrittmachers, mit einem Film der wässrigen Lösung auf ihrer Oberfläche in die vom operierenden Chirurgen vorbereitete Tasche implantiert wurde und die Tasche anschließend mit der restlichen Lösung (ca. 100 ml) gespült wurde, wurden bei einer vergleichbaren Zahl von Implantationen keinerlei Infektionen beobachtet.

Schrittmacherimplantationen unter erfindungsgemäßer Verwendung einer 2,0 Gew.-%igen wässrigen Taurolidinlösung sind in der nachfolgenden Tabelle 1 tabellarisch erfasst. Die Tabelle gibt dabei die Befunde für 45 Implantationen, einschließlich von 9 Fällen des Austauschs eines Aggregats gegen ein neues, wieder, die im Zeitraum zwischen Februar und September 2015 erfolgten, wobei in allen Fällen die angegebene 2,0 Gew.-%ige wässrige Taurolidinlösung wie beschrieben zur Anwendung kam.

In der Tabelle sind die Patienten mit von ihren Vornamen abgeleiteten Initialen und ihrem Geburtsjahr individualisiert. Außerdem sind die Operationsdauer sowie das implantierte Schrittmacher/ICD-System angegeben. Da die einzelnen Implantationen innerhalb eines längeren Zeitraums erfolgten, liegen sie im Zeitpunkt der Einreichung der vorliegenden Anmeldung unterschiedlich lange zurück, d.h. der Überwachungszeitraum ist für die verschiedenen Implantationen etwas unterschiedlich.

Von den Patienten in Tabelle 1 waren 12 Patienten weiblich und 33 Patienten männlich. Das Alter der Patienten lag zwischen 94 Jahren und 47 Jahren.

Bis zum Anmeldetag der vorliegenden Anmeldung wurde - anders als bei den Fällen einer Wasserstoffperoxidbehandlung - bei keinem einzigen der Patienten eine Infektion beobachtet. Aufgrund der erkennbaren Überlegenheit einer Vorgehensweise gemäß der Erfindung wurde die Praxis ganz auf deren Anwendung umgestellt, und aus ethischen Gründen sind keine weiteren Zahlen für Behandlungen mit Wasserstoffperoxid mehr zu erwarten.

Bei der Anwendung der Erfindung wurde zwischen allen Beteiligten Personen bis heute die Wahrung der Vertraulichkeit vereinbart und gewahrt.

Außer dass die erfindungsgemäße Vorgehensweise nach allen vorliegenden Erkenntnissen einen überlegenen Schutz gegen Infektionen (DRIs) bietet, wird auch eine gute, ungestörte Wundheilung beobachtet.

Es liegt auch im Bereich der vorliegenden Erfindung, die erfindungsgemäß verwendbaren wässrigen Taurolidin-Lösungen auch im Rahmen einer Technik einzusetzen, bei der - wie in der eingangs erwähnten Veröffentlichung von J. Alberto Lopez - eine Spülung infizierter Herzschrittmachertaschen erfolgt. Auch wenn die Erfindung in der vorliegenden Anmeldung im Hinblick auf CIEDs beschrieben wird, d.h. auf ein Gebiet, auf dem bereits vorteilhaften klinische Ergebnisse nachgewiesen werden konnten, gehen die Erfinder davon aus, dass sich ähnliche Vorteile im Hinblick auf eine Infektionsvermeidung auch bei anderen herzchirurgisch implantierten Vorrichtungen ohne elektronische bzw. elektrophysiologische Komponenten feststellen lassen, insbesondere bei künstlichen Herzklappen.

**Tabelle 1**

| Patient | Geburtsjahr | Operationsdauer | Implantiertes System* |
|---|---|---|---|
| FR | 1937 | 1 h 42 min | CRT-D-DDD |
| MO | 1958 | 1 h 17 min | DDD II-SM |
| BA | 1945 | 33 min | DDD-SM |
| AN | 1957 | 36 min | VVI_ICD |
| KA | 1933 | 15 min | VVI_ICD |
| FR2 | 1960 | 19 min | VVI_ICD |
| BE | 1936 | 12 min | CRT-D-DDD |
| JO | 1952 | 15 min | DDD-SM |
| MX | 1937 | 32 min | VVI-ICD |
| TH | 1927 | 8 min | DDD-SM |
| JO2 | 1928 | 15 min | DDD-SM |
| KL | 1937 | 31 min | DDD-SM |
| MA | 1953 | 20 min | VVI-ICD |
| MT | 1956 | 115 min | VVI-ICD |
| LU | 1945 | 20 min | VVI-ICD |
| WE | 1943 | 82 min | CRT-D-DDD |
| RO | 1968 | 45 min | DDD-SM |
| JO3 | 1937 | 41 min | DDD-SM |
| PE | 1961 | 28 min | VVI-ICD |
| HE | 1947 | 48 min | DDD-ICD |
| BR | 1939 | 84 min | DDD-ICD |
| JH | 1934 | 25 min | VVI-SM |
| HI | 1923 | 28 min | VVI-SM |
| JH2 | 1938 | 24 min | VVI-SM |
| HN | 1940 | 33 min | DDD+RV Sonde |
| WE2 | 1931 | 17 min | VVI-SM |
| JO4 | 1941 | 85 min | CRT-D-VVI |
| KA2 | 1932 | 60 min | DDD-SM |
| RE | 1948 | 55 min | DDD-SM |
| AD | 1937 | 31 min | VVI-ICD |
| ZB | 1953 | 13 min | VVI-ICD |
| SI | 1962 | 32 min | VVI-ICD |
| FR3 | 1942 | 53 min | VVI-ICD |
| FI | 1937 | 179 min | CRT-D |
| HA | 1938 | 185 min | CRT-D |
| JP | 1966 | 70 min | VVI-ICD |
| KT | 1921 | 22 min | VVI-ICD |
| AL | 1938 | 87 min | VVI-ICD |
| IR | 1944 | 64 min | CRT-P |
| TH2 | 1934 | 110 min | DDD-SM |
| FR4 | 1934 | 76 min | CRT-D |
| MA2 | 1931 | 91 min | DDD-SM |
| SF | 1952 | 92 min | CRT-D |
| FR5 | 1942 | 40 min | DDD-SM |
| HR | 1960 | 29 min | VVI-ICD |

### Erläuterungen zu Tabelle 1:

### Abkürzungen:

- SM: Schrittmacher (Pacemaker)
- ICD: Implantable Cardiac Device
- CRT-D: Cardiac Resynchronisation Therapy - Defibrillator

Zur näheren Kennzeichnung und Unterscheidung verschiedener Vorrichtungstypen wird eine einheitliche Nomenklatur verwendet, bei der die Herzschrittmacher mit einem drei bis fünf Buchstaben umfassenden Kürzel bezeichnet werden:
- Die erste Stelle gibt den Stimulationsort an. Hierbei steht V für Ventrikel (Kammer), A für Atrium (Vorhof) und D für beide.
- Die zweite gibt den Ort der Signalaufnahme an. Die Abkürzungen entsprechen denen für die erste Stelle.
- Die dritte Stelle gibt den Modus an. Hierbei steht I für inhibiert, T für getriggert (auslösend), und D für durch den Vorhof getriggert und die Kammer inhibiert.
- Die vierte Stelle liefert Informationen über die Art der Programmierung, z.B. R für frequenzmoduliert (durch die Vorhoffrequenz variiert).
- Die fünfte Stelle gibt Auskunft über einen möglichen implantierten (enthaltenen) AICD (automatic implantable cardioverter defibrilator.

Typische, häufig benötigte Abkürzungen sind DDD und VVI.

## Patentansprüche

1. Wässrige Lösung von Kondensationsprodukten von Taurinamid und Methylenglykol und deren Salzen, die im wesentlichen nur Taurolidin und/oder dessen Hydrolyseprodukte in einer Konzentration von 0,5 bis 3 Gew.-% oder gelöstes Taurultam in einer Konzentration von 1 bis 7,5 Gew.-% sowie außerdem als Lösungsvermittler und/oder die Benetzung von Metall- und Kunststoffoberflächen förderndes Mittel ein wassermischbares, physiologisch verträgliches Polymer in Form von Polyvinylpyrrolidon (PVP, Povidon) mit einem mittleren Molekulargewicht von weniger als 30.000 enthält, das in der Lösung in Mengen von 3 bis 7 Gew.% vorhanden ist,
zur Verwendung als antiinfektives Mittel bei der Implantation einer implantierbaren elektronischen Vorrichtung in der Herzchirurgie nach einem Verfahren, bei dem man die wässrige Lösung zur Oberflächenbehandlung der implantierbaren elektronischen Vorrichtung vor der Implantation verwendet *und die implantierbare elektronische Vorrichtung mit einem Film der wässrigen Lösung auf ihrer Oberfläche implantiert.*

2. Wässrige Lösung nach Anspruch 1 zur Verwendung gemäß Anspruch 1, **dadurch gekennzeichnet, dass** sie durch Auflösen von Taurolidin, Taurultam oder Cyclotaurolidin hergestellt wurde.

3. Wässrige Lösung nach Anspruch 1 zur Verwendung gemäß Anspruch 1, **dadurch gekennzeichnet, dass** das wassermischbare Polymer Polyvinylpyrrolidon (PVP, Povidon) mit einem mittleren Molekulargewicht von weniger als 10.000, beispielsweise zwischen 7000 und 9000, ist und in der Lösung in Mengen von 3 bis 7 Gew.-%, beispielsweise von 5 Gew.-%, vorhanden ist.

4. Wässrige Lösung nach einem der Ansprüche 1 bis 3 zur Verwendung gemäß Anspruch 1, **dadurch gekennzeichnet, dass** die implantierbare elektronische Vorrichtung ausgewählt ist aus Herzschrittmachern, implantierbaren Kardioverter-Defibrillatoren, kardialen Resynchronisationstherapie-Defibrillatoren, subkutan implantierbaren Kardioverter-Defibrillatoren und implantierbaren elektronischen Überwachungsvorrichtungen für diagnostische Zwecke.

5. Wässrige Lösung nach einem der Ansprüche 1 bis 4 zur Verwendung gemäß Anspruch 1, bei der die implantierbare elektronische Vorrichtung vor der Implantation für die zur Erzielung der gewünschten antimikrobiellen Wirkung und vollständigen Benetzung aller Oberflächen der Vorrichtung erforderliche Zeit in die Lösung eingelegt wird und die implantierbare Vorrichtung mit einem Film der wässrigen Lösung auf ihrer Oberfläche implantiert wird.

## Claims

1. Aqueous solution of condensation products of taurinamide and methylene glycol and the salts thereof, which substantially contains only taurolidine and/or the hydrolysis products thereof in a concentration of 0.5 to 3 wt% or dissolved taurultam in a concentration of 1 to 7,5 wt% and, in addition, a water-miscible, physiologically tolerated polymer in the form of polyvinyl pyrrolidone (PVP, povidone) having an average molecular weight of less than 30,000 as a solubilizing agent and/or an agent promoting the wetting of metal and plastic surfaces, which is present in the solution in amounts of 3 to 7 wt%,
for use as an anti-infective agent in the implantation of an implantable electronic device in cardiac surgery according to a method in which the aqueous solution is used for the surface treatment of the implantable electronic device prior to implantation *and the implantable electronic device is implanted with a film of an aqueous solution on the surface thereof.*

2. Aqueous solution according to claim 1 for use according to claim 1, **characterized in that** it has been prepared by dissolving taurolidine, taurultam or cyclotaurolidine.

3. Aqueous solution according to claim 1 for use according to claim 1, **characterized in that** the water-miscible polymer is polyvinyl pyrrolidone (PVP, povidone) having an average molecular weight of less than 10,000, for example between 7,000 and 9,000, and is present in the solution in amounts of 3 to 7 wt%, for example 5 wt%.

4. Aqueous solution according to any one of claims 1 to 3 for use according to claim 1, **characterized in that** the implantable electronic device is selected from pacemakers, implantable cardioverter defibrillators, cardiac resynchronization therapy defibrillators, subcutaneously implantable cardioverter defibrillators and implantable electronic monitoring devices for diagnostic purposes.

5. Aqueous solution according to any of claims 1 to 4 for use according to claim 1, wherein the implantable electronic device is placed in the solution prior to implantation for the time required to achieve the desired antimicrobial effect and complete wetting of all surfaces of the device, and the implantable device is implanted with a film of the aqueous solution on the surface thereof.

## Revendications

1. Solution aqueuse de produits de condensation de taurinamide et de méthylène glycol et de leurs sels, qui contient sensiblement uniquement de la taurolidine et/ou ses produits d'hydrolyse dans une concentration de 0,5 à 3 % en poids ou du taurultame dissous dans une concentration de 1 à 7,5 % en poids ainsi que, en tant qu'agent solubilisant et/ou agent favorisant le mouillage de surfaces métalliques et plastiques, un polymère physiologiquement compatible, miscible à l'eau, sous la forme de polyvinylpyrrolidone (PVP, povidone) ayant un poids moléculaire moyen inférieur à 30 000, qui est présent dans la solution dans des quantités de 3 à 7 % en poids,
destinée à être utilisé comme agent anti-infectieux lors de l'implantation d'un dispositif électronique implantable en chirurgie cardiaque selon un procédé dans lequel on utilise la solution aqueuse pour le traitement de surface du dispositif électronique implantable avant l'implantation et on implante le dispositif électronique implantable avec un film de la solution aqueuse sur sa surface.

2. Solution aqueuse selon la revendication 1, destinée à être utilisée selon la revendication 1, **caractérisée en ce qu'**elle a été préparée par dissolution de taurolidine, taurultame ou cyclotaurolidine.

3. Solution aqueuse selon la revendication 1, destinée à être utilisée selon la revendication 1, **caractérisée en ce que** le polymère miscible à l'eau est de la polyvinylpyrrolidone (PVP, povidone) ayant un poids moléculaire moyen inférieur à 10 000, par exemple compris entre 7 000 et 9 000, et est présent dans la solution dans des quantités de 3 à 7 % en poids, par exemple de 5 % en poids.

4. Solution aqueuse selon l'une des revendications 1 à 3, destinée à être utilisée selon la revendication 1, **caractérisée en ce que** le dispositif électronique implantable est choisi parmi les stimulateurs cardiaques, les défibrillateurs cardioverteurs implantables, les défibrillateurs de thérapie de resynchronisation cardiaque, les défibrillateurs cardioverteurs implantables sous la peau et les dispositifs électroniques de surveillance implantables à des fins de diagnostic.

5. Solution aqueuse selon l'une des revendications 1 à 4, destinée à être utilisée selon la revendication 1, de telle sorte que, avant son implantation, le dispositif électronique implantable est placé dans la solution pendant le temps nécessaire pour obtenir l'effet antimicrobien souhaité et le mouillage complet de toutes les surfaces du dispositif, et le dispositif implantable est implanté avec un film de la solution aqueuse sur sa surface.
